# EUROPÄISCHE PATENTSCHRIFT

(11) **EP 0 668 050 B1**
(45) Veröffentlichungstag und Bekanntmachung des Hinweises auf die Patenterteilung: **07.07.1999**
(21) Anmeldenummer: 95101505.6
(22) Anmeldetag: 03.02.1995
(51) Int. Cl.: A61B 6/14

(54) **Zahnärztliche Röntgendiagnostikeinrichtung zur Erstellung von Panorama-Röntgenaufnahmen vom Schädel eines Patienten**
Dental diagnostic x-ray apparatus providing panorama for patient's head
Appareil de radiodiagnistic dentaire panoramique du crâne

(30) Priorität: 14.02.1994 DE 4404640
(43) Veröffentlichungstag der Anmeldung: 23.08.1995
(73) Patentinhaber: Sirona Dental Systems GmbH, 64625 Bensheim (DE)
(72) Erfinder: Günther, Werner, D-64625 Bensheim (DE); Molitor, Dieter, D-68642 Bürstadt (DE); Werner, Leonhard, Dipl.-Ing. (FH), D-69502 Hemsbach (DE)
(74) Vertreter: Isenbruck, Günter, Dr.

(56) Entgegenhaltungen:
- EP-A- 0 204 676
- DE-A- 3 703 050
- FR-A- 2 645 007
- US-A- 2 818 510
- US-A- 5 058 147
- US-A- 5 148 454
- US-A- 5 214 686

## Beschreibung

Die Erfindung bezieht sich auf eine zahnärztliche Röntgendiagnostikeinrichtung, wie sie beispielsweise aus der EP-0 229 308 oder US-PS 4 811 372 bekannt ist. Eine solche Einrichtung enthält eine um den Patientenkopf herum bewegbare Dreheinheit, an der diametral einander gegenüber eine Röntgenstrahlenquelle und eine Röntgenstrahlenaufnahmeeinheit gehaltert sind. Bei der bekannten Einrichtung ist die Dreheinheit ringförmig ausgebildet und an einer Standsäule höhenverstellbar gehaltert. Die Dreheinheit enthält einen drehbaren Teil, an dem Strahlenquelle und Aufnahmeeinheit (Filmkassette) gehaltert sind und einen demgegenüber festen Trägerteil, der an der Standsäule höhenverstellbar und außerdem noch um einen vom Drehzentrum abweichenden Punkt schwenkbar angelenkt ist. Die Einrichtung enthalt ferner in bekannter Weise eine den Patientenkopf in reproduzierbarer Weise fixierende Positioniereinheit, die in der Regel aus einem Stirn und Schläfe des Patienten fixierbaren Schädelhalter mit Aufbißteil oder Kinnstütze besteht.

Bei einer anderen bekannten Röntgeneinrichtung (DE-A1-37 03 050), die vornehmlich dazu ausgebildet ist, Röntgenaufnahmen von einzelnen Bereichen des Zahn-, Kiefer- und Schädelbereiches erstellen zu können, enthält die an einer Säule über einen auslegeartigen Träger um eine lotrechte Achse schwenkbare Dreheinheit einen rotierenden Abschnitt, der mit dem Träger über eine vertikale Achse verbunden ist. Der rotierende Abschnitt trägt einen bogenförmigen Träger, an dessen Enden einerseits die Röntgenstrahlenquelle und andererseits eine Filmkassettenhalterung angeordnet sind.

Ausgehend von der bekannten Röntgendiagnostikeinrichtung liegt der im Anspruch 1 angegebenen Erfindung die Aufgabe zugrunde, demgegenüber eine preisgünstigere und platzsparendere Röntgeneinrichtung anzugeben.

Die Erfindung fußt auf der Erkenntnis, daß man vorzugsweise bei sitzendem Patienten alleine durch eine Schwenkbewegung der auslegeartig z.B. an einer Wand gehalterten Dreh- und Positioniereinheit praktisch alle Patientenkörpergrößen erfassen kann. Es wurde herausgefunden, daß bei sitzendem Patienten die Unterschiede zwischen kleinen Patienten mit einer Körpergröße von etwa 1.50 m und großen Patienten mit einer Körpergröße von etwa 2 m die Verstellunterschiede für die verschwenkbare Dreheinheit nur etwa 100 bis 150 mm betragen. Unter Einbeziehung selbst von kleinen Kindern beträgt der Unterschied maximal nur 250 mm. Diesen Verstellweg kann man durch eine Schwenkbewegung der Dreh- und Positioniereinheit durchführen, ohne daß dies für den Patienten hinderlich ist. Durch das Wegschwenken der Dreh- und Positioniereinheit bei Nichtgebrauch wird insgesamt ein extrem kleiner Stellplatz für die gesamte Einrichtung benötigt. Die Einrichtung läßt sich also insbesondere bei sehr kleinen Raumverhältnissen unterbringen.

Durch die Koppelung mit einem Röntgendiagnostikgerät zur Erstellung von sogenannten Intraoralaufnahmen, d.h. mit intraoral plazierbarer Röntgenaufnahmeeinheit (Film oder Sensor), läßt sich bei gemeinsamer Nutzung der vorzusehenden Netzteile und Steuerelektronik eine universellere Nutzung erzielen. Durch diese Maßnahmen läßt sich in platzsparender und auch techhnisch einfacherer und damit kostensparender Weise eine komplette Röntgenstation für Einzelzahnaufnahmen und für Panoramaaufnahmen des Kiefers erstellen.

Weitere Vorteile ergeben sich aus den Patentansprüchen und der nachfolgenden Beschreibung mehrerer Ausführungsbeispiele der Erfindung. Es zeigen:
Figur 1 die erfindungsgemäße Röntgendiagnostikeinrichtung in einer ersten Ausbaustufe in Frontansicht,
Figur 2 die Einrichtung gemäß Figur 1 in perspektivischer Darstellung in einer ersten Benutzerposition,
Figur 3 die Einrichtung wie in Figur 1 gezeigt in einer zweiten Benutzerposition,
Figur 4 eine Prinzipdarstellung einer ähnlich in Figur 3 gezeigten Benutzerposition,
Figuren 5 und 6 Einzelheiten von dem an der Wand befestigbaren Gehäuse,
Figur 7 ein Prinzipblockschaltbild der Einrichtung,
Figur 8 eine weitere Variante einer Einrichtung nach der Erfindung in schaubildlicher Darstellung.

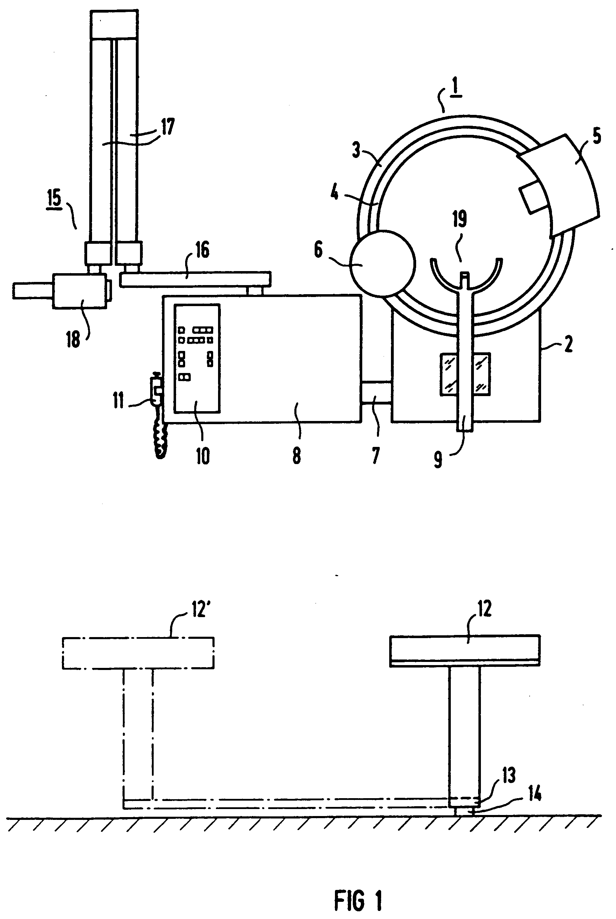

Die Figur 1 zeigt in einer Frontansicht die erfindungsgemäße Einrichtung in einer ersten Ausbaustufe. Die Grundausstattung beinhaltet zunächst nur ein Röntgengerät zur Erstellung von Panorama-Schichtaufnahmen vom Schädel eines Patienten. Dieses Röntgengerät enthält eine Dreheinheit 1, welche in bekannter Weise einen mit einem Träger 2 verbundenen ersten (äußeren) Ring 3 aufweist, an dem ein demgegenüber drehbar angeordneter zweiter (innerer) Ring 4 gehaltert ist. Am zweiten Ring 4 sind diametral gegeneinander gegenüberliegend eine Röntgenstrahlenquelle 5 sowie eine Röntgenstrahlenaufnahmeeinheit 6 gehaltert. Die Aufnahmeeinheit 6 kann, wie in der Figur gezeigt, eine in Rotation versetzbare Filmtrommel sein, die eine flexible Filmkassette aufweist; bei einer digitalen Röntgeneinrichtung kann anstelle der Filmtrommel auch ein CCD-Zeilensensor vorgesehen sein. Während einer Panoramaufnahme wird der zweite Ring 4 um seine Mitttelpunktachse gedreht, d.h. Strahler und Filmkassette umkreisen den Patientenkopf, während der erste Ring 3 um einen außerhalb des Mittelpunkts befindlichen Drehpunkt geschwenkt wird. Die Steuerung der erforderlichen Antriebe, um eine tomographische Aufnahme erstellen zu können, ist bekannt, so daß hierauf nicht näher eingegangen zu werden braucht.

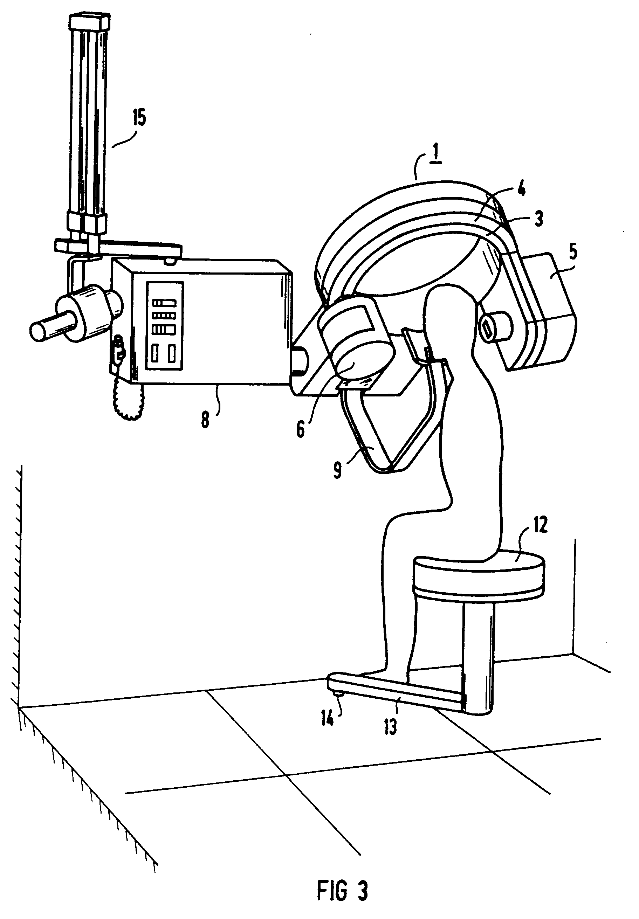

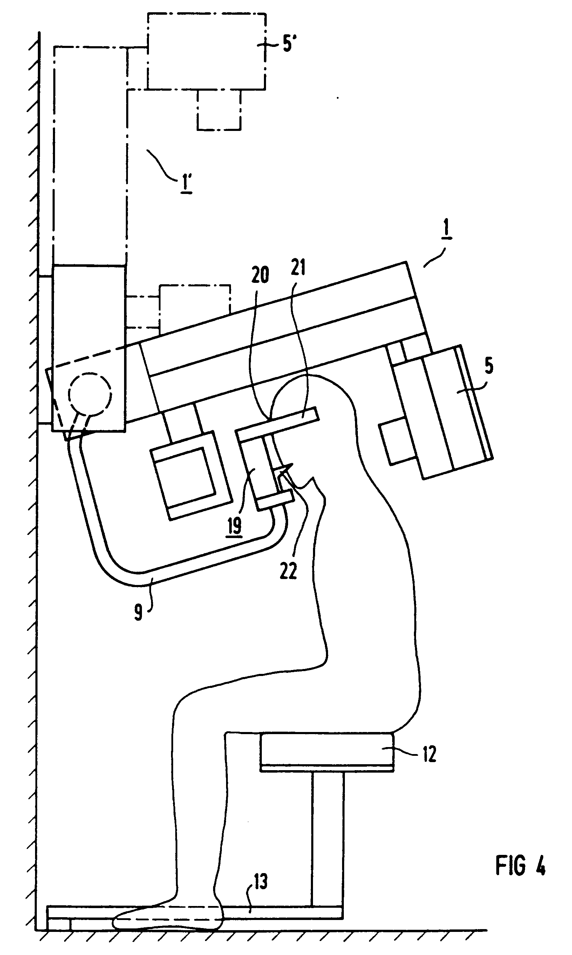

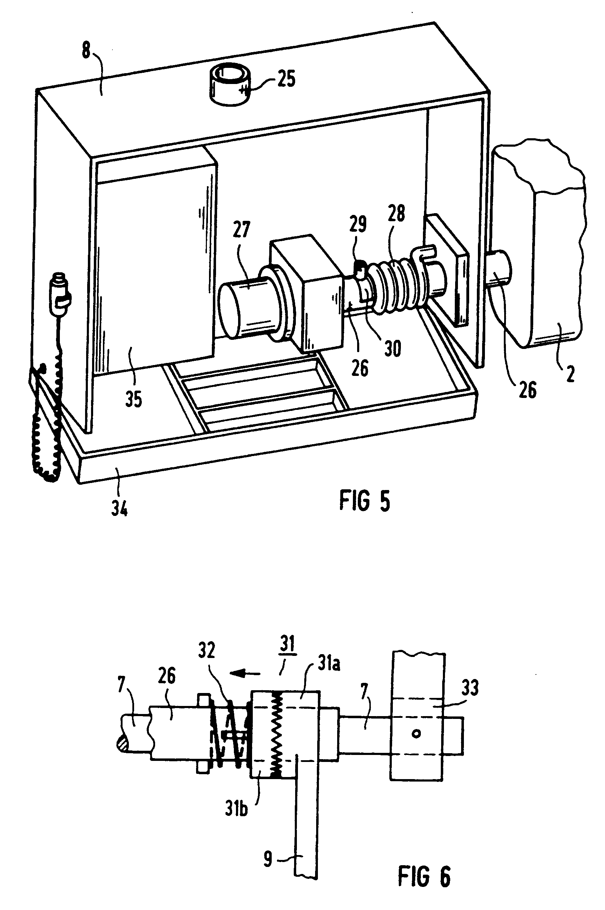

Die Dreheinheit 1 ist in der Figur in einer Nichtgebrauchsstellung gezeigt, in der Strahlenquelle und Aufnahmeeinheit in einer vertikalen Ebene, also parallel zur Wand, zu liegen kommen. Mittels einer Schwenkachse 7 kann die Dreheinheit 1 in verschiedene Benutzerpositionen gebracht werden (Fig. 3 und 4). Die Schwenkachse 7 ist hierzu in einem an einer Wand befestigbaren Gehäuse 8 entsprechend gelagert. Näheres hierzu ergibt sich aus den Figuren 5 und 6 mit zugehöriger Beschreibung.

Gleichachsig mit der Schwenkachse 7, jedoch getrennt von dieser verstellbar, ist im Träger 2 eine Positioniereinheit 9 schwenkbar gelagert. Die Positioniereinheit 9 enthält geeignete Mittel zur reproduzierbaren Fixierung des Patientenkopfes in bezug auf die Gesichtsmitte und die Frankfurter Horizontale. Als Einstellhilfe dient ein nicht näher bezeichneter Spiegel am Träger. Die vorgenannten Mitttel sind vorteilhafterweise ein visierartig den Patientenkopf frontseitig umgebender Schädelhalter 19 aus Plexiglas oder ähnlichem durchsichtigem Material, an dem eine verstellbare Stirnstütze 20, mit an den Patientenkopf seitlich anlegbaren Schläfenstützen 21 sowie ein auswechselbares Aufbißteil 22 zur Fixierung der Frontzähne des Patienten gehaltert sind (Fig. 4).

Das Gehäuse 8 enthält frontseitig ein Anzeige- und Bedienfeld 10 mit diversen Schalt- und/oder Anzeigeelementen. Mit 11 ist ein Auslöseschalter bezeichnet, mit dem von einer Bedienperson der betreffende Röntgenstrahler aktiviert und damit eine Aufnahme ausgelöst werden kann.

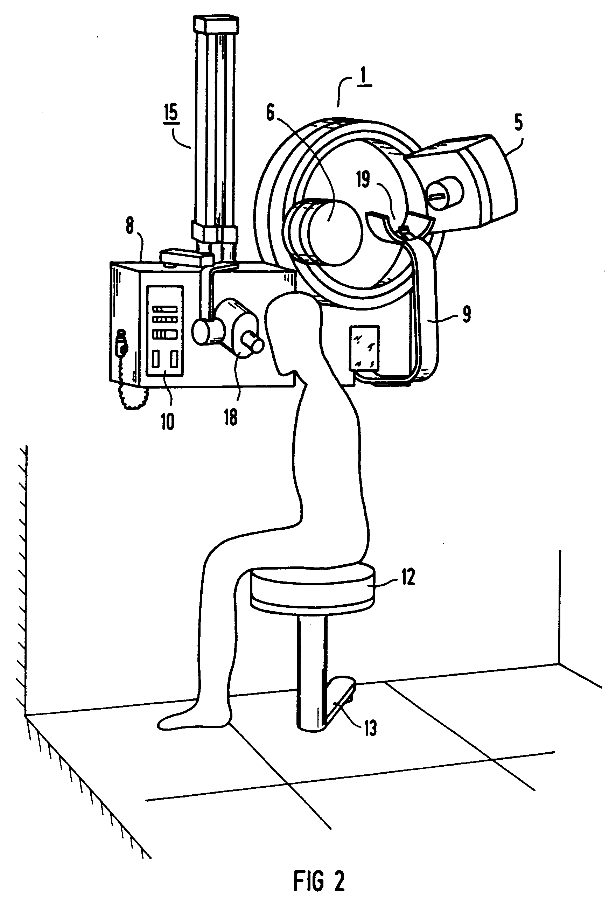

Dem vorbeschriebenen Röntgengerät ist ein Patientenstuhl 12 zugeordnet, der mittels eines Schwenkarmes 13 und einer wandnah befindlichen Achslagerung 14 in bezug auf das Röntgengerät so angeordnet ist, daß in der Benutzerposition ein auf dem Stuhl sitzender Patient in bezug auf die Dreheinheit 1 positionierbar ist. Wie aus der strichpunktierten Darstellung ersichtlich, kann der Stuhl um die Achslagerung 14 in eine Schwenkposition (12') gebracht werden, in der gemäß der bereits erwähnten Ausbaustufe mit Hilfe eines ebenfalls am Gehäuse 8 gehalterten zweiten Röntgengerätes 15 sogenannte Intraoralaufnahmen gemacht werden können. Eine mögliche Benutzerposition für eine solche Intraoralaufnahme ist in Figur 2 schaubildlich dargestellt.
Das am Gehäuse 8 gehalterte Röntgengerät 15 enthält einen Schwenkarm 16, an dem ein Doppelgelenkarm 17 angelenkt ist, an dessen freiem Ende in bekannter Weise eine Röntgenstrahlenquelle 18 angelenkt ist. Wenn in der in Figur 2 dargestellten Benutzerposition Intraoralaufnahmen gemacht werden, befindet sich die Dreheinheit 1 des erstgenannten Röntgengerätes in den Figuren 1 und 2 dargestellten Nichtgebrauchs stellungen.

Zur Erstellung von Panoramaaufnahmen kann die Dreheinheit aus der in Figur 1 und 2 gezeigten Nichtgebrauchsstellung in verschiedene Benutzerpositionen gebracht werden, wie beispielsweise in den Figuren 3 und 4 dargestellt. Dreheinheit 1 und Positioniereinheit 9 werden hierzu um einen bestimmten Winkel geschwenkt. Der Schwenkwinkel ist durch die Kopfhöhe des zu diagnostizierenden Patienten vorgegeben, wenn er auf dem Patientenstuhl 12 Platz genommen hat. Zur Vorbereitung einer Aufnahme wird zunächst die Positioniereinheit 9 so weit geschwenkt, bis der Patientenkopf im Schädelhalter 19, exakt positionierbar ist. Danach wird die Dreheinheit 1 ebenfalls geschwenkt, und zwar so weit, bis diese in einer festen Zuordnung zur Positioniereinheit bzw. zum Schädelhalter steht. Diese Zuordnung von Dreh- und Positioniereinheit ist bei jeder Patienteneinstellung gegeben, so daß, gleichgültig, ob ein großer oder kleiner Patient auf dem Stuhl Platz genommen hat, stets eine exakte Durchstrahlung des Schädels entsprechend des zuvor fixierten Patientenkopfes gegeben ist. Die Positioniereinheit 9 kann in der jeweils dem Patienten angepaßten Schwenkstellung z.B. durch eine Magnetbremse oder eine andere geeignete Einrichtung fixiert werden. Hierzu können mechanische und/oder elektrische Kupplungs- und Rastmittel vorgesehen sein, wie dies im Ausführungsbeispiel in Figur 6 dargestellt ist.

Die Figur 4 zeigt in einer vereinfachten Darstellung die Dreheinheit in einer Seitenansicht. Aus der Darstellung ist einerseits die Nichtgebrauchsstellung (strichpunktierte Linienführung) und andererseits eine von mehreren möglichen Benutzerpositionen sowie die Zuordnung zum Patienten erkennbar.

Die Figur 5 zeigt in schaubildlicher Darstellung das Gehäuse 8 bei abgenommener Frontwand. An der Oberseite des Gehäuses befindet sich eine Lagerbuchse 25 zur Aufnahme des Schwenkarmes 16. Die Schwenkachse 7, welche mit dem Träger 2 drehfest verbunden ist, ist in einem Lagerrohr 26 drehbar gelagert und kann mit Hilfe eines schematisiert dargestellten motorischen Antriebes 27 gedreht werden. Der Antrieb 27 kann vorteilhafterweise aus einem Schrittmotor und einem entsprechenden Übersetzungsgetriebe bestehen. Konzentrisch zur Schwenkachse 7 ist auf dem Lagerrohr 26 eine Torsionsfeder 28 angeordnet, deren eines Ende über einen Mitnehmer 29 mit der Schwenkachse verbunden ist und deren anderes Ende sich am Gehäuse 8 abstützt. Die Torsionsfeder dient zur Gewichtskompensation und ist so bemessen, daß die gesamte Dreheinheit in jeder Schwenkstellung gewichtsausgeglichen ist. Der Antrieb 27 kann dadurch sehr klein dimensioniert werden.

Der Mitnehmer 29 ist an der Schwenkachse 7 befestigt und greift durch einen Schlitz 30 im Lagerrohr 26; er klemmt einerseits das erwähnte eine Ende der Torsionsfeder 28 an der Schwenkachse 7 fest und bildet andererseits mit den Schlitzenden einen Anschlag.

Das Lagerrohr 26 reicht bis in das Gehäuse des Trägers 2 hinein und enthält eine rastbare Kupplung 31 für die Positioniereinheit 9., die in Figur 6 in einer mechanischen Ausführung dargestellt ist. Die Kupplung 31 besteht aus zwei Zahnscheiben 31a und 31b, von denen die eine (31a) drehbar am Lagerrohr angeordnet und mit der Positioniereinheit 9 fest verbunden ist und die andere (31b) drehfest, jedoch axial am Lagerrohr geführt und mit Hilfe eines nicht dargestellten Ausrückhebels entgegen der Kraft einer Feder 32 in Pfeilrichung ausrückbar angeordnet ist. Zum Einstellen der Positioniereinheit auf den Patienten wird zunächst die Zahnscheibe 31b in Pfeilrichtung ausgerückt, wodurch die Positioniereinheit 9 in eine auf den Patienten angepaßte Benutzerposition gebbracht werden kann. Danach kann die Dreheinheit 1 mittels des Antriebs 27 auf diese Position eingestellt werden. Hierzu ist der Träger 2 über einen z.B. gabelförmigen Mitnehmer 33 mit der Achse 7 verbunden.

Der Gehäuseboden ist vorteilhafterweise als abklappbare Lade 34 ausgebildet und mit diversen Unterteilungen versehen, so daß darin den Röntgengeräten zugeordnete Zubehörteile oder dergleichen aufbewahrt werden können.

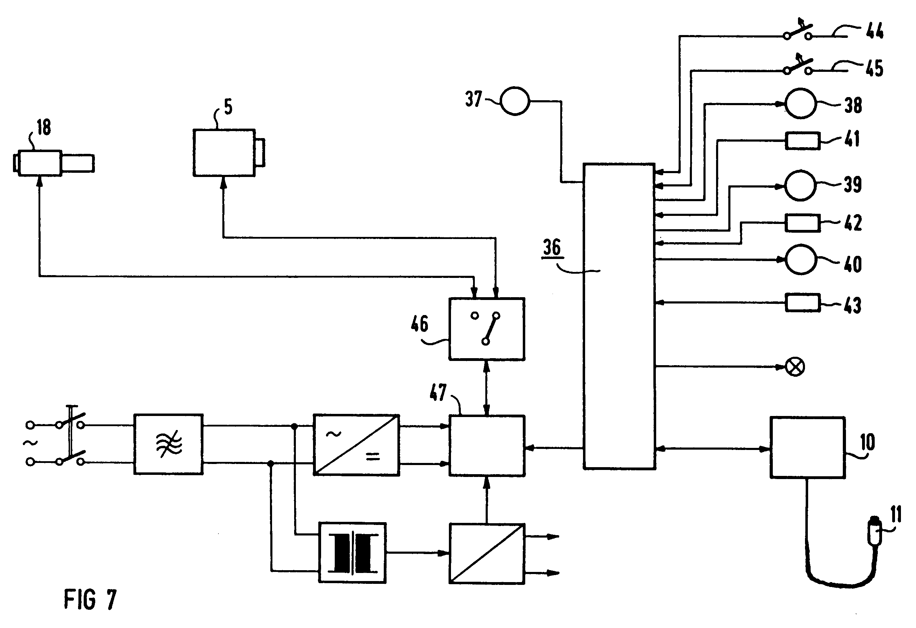

Im Innern des Gehäuses 8 ist ferner ein Steuerteil 35 untergebracht, der entsprechend dem Blockschaltbild nach Figur 7 die Elektronik zur Ansteuerung der vorhandenen Antriebe sowie der beiden Röntgenstrahlenquellen enthält.

Die Figur 7 zeigt ein Blockschaltbild für die zuvor beschriebene Anlage. Das Steuerteil 35 enthält eine zentrale Steuerelektronik 36 mit einem Mikroprozessor, über den vier Verstellmotore 37 bis 40 gesteuert werden. Der erste dieser Verstellmotore dient zur Neigung der gesamten Dreheinheit, der zweite veranlaßt den Umlauf des drehbaren Ringes, der dritte bewirkt den Umlauf der Filmkassette und der vierte dient für die erwähnte Schwenkbewegung des nicht drehbaren Teils des Ringes in horizontaler Ebene. Die Verstellmotore sind vorteilhafterweise Schrittmotore.

Mit 41 bis 43 sind Lagesensoren bezeichnet, die Information über die Startposition, des Drehringes, der Filmkassette und der Schwenkstellung des Drehringes geben. Diese Lagesensoren können beispielsweise Gabellichtschranken sein. Die Nichtgebrauchsstellung sowie die Benutzerposition der Dreheinheit wird durch Mikroschalter oder Gabellichtschranken 44 und 45 erfaßt, die unterschiedliche Ansteuerung der Strahlenguellen 5 und 18 erfolgt mittels eines Umschalters 46, der den Ausgang eines Wechselrichters 47, je nachdem, welches Röntgengerät zum Einsatz kommt, mit der Strahlenquelle 5 oder 18 verbindet. Das Umschalten kann automatisch mit den Schaltern 44 und 45 erfolgen oder auch von Hand durchgeführt werden.

Die übrigen Bauteile des Steuerteils, wie Netzschalter, Netzgleichrichter, Spannungsumschalter und Spannungswandler, sind, da üblich, nicht näher bezeichnet.

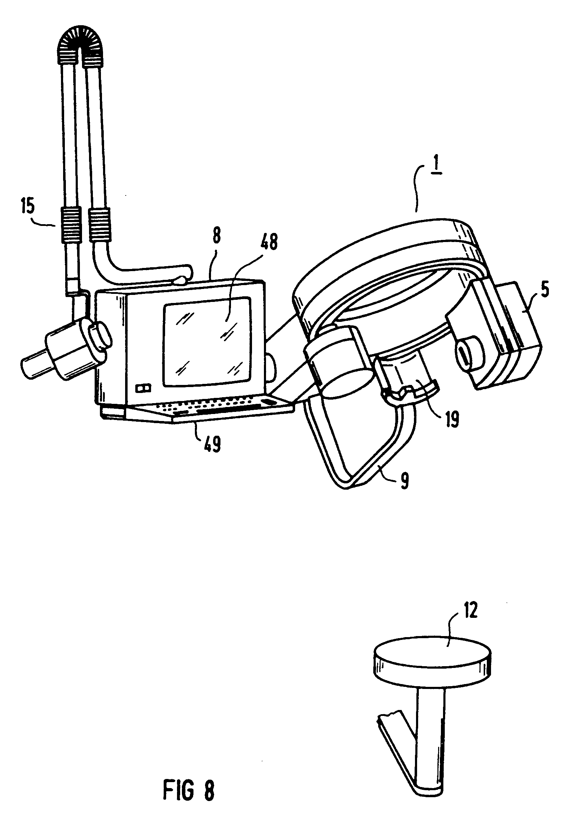

Die Figur 8 zeigt eine weitere Ausbaustufe der erfindungsgemäßen Einrichtung. Bei dieser Variante enthält das Gehäuse 8 frontseitig einen Flachbildschirm 48 und eine Eingabetastatur 49. Eine solche Kombination ist insbesondere von Vorteil bei einem Röntgengerät mit digitaler Bildverarbeitung, bei der anstelle einer Filmkassette ein röntgenstrahlenempfindlicher Sensor mit entsprechender Bildverarbeitung vorgesehen ist.

## Patentansprüche

1. Zahnärztliche Röntgendiagnostikeinrichtung zur Erstellung von Panorama-Röntgenaufnahmen vom Schädel eines Patienten, enthaltend eine um den Patientenkopf bewegbare Dreheinheit (1), an der diametral einander gegenüber eine Röntgenstrahlenquelle (5) und eine Röntgenstrahlenaufnahmeeinheit (6) gehaltert sind sowie eine den Patientenkopf in reproduzierbarer Weise fixierende Positioniereinheit (9), wobei Dreheinheit (1) und Positioniereinheit (9) auslegerartig an einem Träger angeordnet sind und die Dreheinheit um eine horizontale Achse schwenkbar und in einzelnen Schwenkstellungen feststellbar ist, **dadurch gekennzeichnet,** daß die Dreheinheit (1) und die Positioniereinheit (9) um eine gemeinsame Achslagerung, deren Schwenkachse (7) parallel zur Ebene Dreheinheit (1) liegt, getrennt Schwenkbar und jeweils in den einzelnen Schwenkstellungen feststellbar angeordnet sind, wobei die einzelnen Schwenkstellungen unterschiedlichen Körpergrößen eines vorzugsweise sitzenden Patienten entsprechen, indem zunächst die Positioniereinheit (9) auf den Patienten und danach die Dreheinheit (1) in fester Zuordnung zur Positioniereinheit (9) einstellbar ist.

2. Zahnärztliche Röntgendiagnostikeinrichtung nach Anspruch 1, **dadurch gekennzeichnet,** daß die Dreheinheit (1) aus einer Nichtgebrauchsstellung, in der Strahlenquelle (5) und Aufnahmeeinheit (6) in einer Ebene liegen, die im wesentlichen senkrecht und parallel zur Dreiheinheit (1) verläuft, in mehrere Gebrauchsstellungen bringbar ist, in denen die durch Strahlenquelle (5) und Aufnahmeeinheit (6) gebildete Ebene einen spitzen Winkel zur vorgenannten senkrechten Ebene bildet.

3. Zahnärztliche Röntgendiagnostikeinrichtung nach Anspruch 1, **dadurch gekennzeichnet,** daß die Schwenkachse (7) von einem vorzugsweise an einer Wand montierbarem Gehäuse (8) getragen wird, in welchem Mittel zur Ansteuerung der Strahlenquelle (5) und zur Steuerung der Bewegung der Dreheinheit (1) untergebracht sind.

4. Zahnärztliche Röntgendiagnostikeinrichtung nach Anspruch 1, **dadurch gekennzeichnet,** daß die Dreheinheit (1) drehfest mit der Schwenkachse (7) verbunden ist und letztere drehbar im Gehäuse (8) gelagert ist.

5. Zahnärztliche Röntgendiagnostikeinrichtung nach Anspruch 4, **dadurch gekennzeichnet,** daß zur Gewichtskompensation der Dreheinheit (1) die Schwenkachse (7) mit einer Torsionsfeder (28) versehen ist, die sich einerseits an der Schwenkachse (7) und andererseits am Gehäuse (8) abstützt.

6. Zahnärztliche Röntgendiagnostikeinrichtung nach Anspruch 3, **dadurch gekennzeichnet,** daß das Gehäuse (8) bodenseitig mit einer ausziehbaren oder abklappbaren Lade (34) zur Aufnahme von Zubehörteilen versehen ist.

7. Zahnärztliche Röntgendiagnostikeinrichtung nach Anspruch 3, **dadurch gekennzeichnet,** daß das Gehäuse (8) mit einem Lagermittel (25) zur schwenkbaren Halterung eines Röntgengerätes (15) zur Erstellung von Intraoralaufnahmen versehen ist.

8. Zahnärztliche Röntgendiagnostikeinrichtung nach Anspruch 7, **dadurch gekennzeichnet,** daß für das Röntgengerät zur Erstellungen der Intraoralaufnahmen und für die Röntgendiagnostikeinrichtung zur Erstellung der Panorama-Röntgenaufnahmen ein gemeinsamer Patientenstuhl (12) vorgesehen ist, der von einem bodenseitig gelagerten Schwenkarm (13) getragen wird.

9. Zahnärztliche Röntgendiagnostikeinrichtung nach Anspruch 8, **dadurch gekennzeichnet,** daß zur Ansteuerung der Strahlenquellen (5, 18) des Röntgengerätes und der Röntgendiagnostikeinrichtung ein gemeinsamer Steuerteil (35) vorhanden ist.

10. Zahnärztliche Röntgendiagnostikeinrichtung nach Anspruch 9, **dadurch gekennzeichnet,** daß ein Umschalter (46) vorhanden ist, mit dem eine Steuer-Elektronik (36) in Abhängigkeit von der Position der Geräte auf die eine (5) oder andere (18) Strahlenquelle umschaltbar ist.

11. Zahnärztliche Röntgendiagnostikeinrichtung nach einem der Ansprüche 1 bis 10, **dadurch gekennzeichnet,** daß die Postioniereinheit (9) eine visierartig ausgebildete Kopfhalterung (19) enthält, an welcher eine verstellbare Stirnanlage (20) an den Patientenkopf anlegbare Schläfenstützen (21) sowie ein Aufbißteil (22) zur Fixierung der Frontzähne des Patienten angeordnet sind.

12. Zahnärztliche Röntgendiagnostikeinrichtung nach einem der Ansprüche 1 bis 11, **dadurch gekennzeichnet,** daß das Röntgengerät zur Erstellung der Intraoralaufnahmen einen digitalen Bildaufnehmer und eine entsprechende Bildauswerteeinrichtung enthält, und das Gehäuse (8) Träger einer Eingabe- und/oder Bildwiedergabeeinrichtung (48, 49) ist.

13. Zahnärztliche Röntgendiagnostikeinrichtung nach Anspruch 4, **dadurch gekennzeichnet,** daß an der Schwenkachse (7) drehfest, jedoch axial verschiebbar, der eine Teil (31b) einer Kupplung (31) angeordnet ist, deren anderer Teil (31a) mit der Positioniereinheit (9) verbunden ist.

## Claims

1. Dental X-ray diagnostic installation for producing panoramic X-ray images of the skull of a patient, containing the rotary unit (1) which can be moved around the patient's head and on which there are held diametrically opposite one another an X-ray source (5) and an X-ray pick-up unit (6) as well as a positioning unit (9) which fixes the patient's head in a reproducible way, it being the case that the rotary unit (1) and positioning unit (9) are arranged in the manner of booms on a support and the rotary unit can be swivelled about a horizontal axis and can be fixed in individual swivel positions, characterized in that the rotary unit (1) and the positioning unit (9) are arranged such that they can be swivelled separately about a common axial bearing whose swivelling shaft (7) is parallel to the plane of the rotary unit (1) and that they can be fixed in each case in the individual swivel positions, the individual swivel positions corresponding to different heights of a preferably seated patient by virtue of the fact that firstly the positioning unit (9) can be adjusted to the patient, and thereafter the rotary unit (1) can be set permanently assigned to the positioning unit (9).

2. Dental X-ray diagnostic installation according to Claim 1, characterized in that the rotary unit (1) can be brought from an idle position, in which the X-ray source (5) and pick-up unit (6) lie in a plane which runs essentially vertical and parallel to the rotary unit (1), into a plurality of positions of use in which the plane formed by the X-ray source (5) and pick-up unit (6) forms an acute angle with the abovementioned vertical plane.

3. Dental X-ray diagnostic installation according to Claim 1, characterized in that the swivelling shaft (7) is supported by a housing (8) which can preferably be mounted on a wall and in which means are accommodated for driving the X-ray source (5) and for controlling the movement of the rotary unit (1).

4. Dental X-ray diagnostic installation according to Claim 1, characterized in that the rotary unit (1) is connected securely in terms of rotation with the swivelling shaft (7), and the latter is mounted rotatably in the housing (8).

5. Dental X-ray diagnostic installation according to Claim 4, characterized in that to compensate the weight of the rotary unit (1) the swivelling shaft (7) is provided with a torsion spring (28) which is supported on the swivelling shaft (7), on the one hand, and on the housing (8), on the other hand.

6. Dental X-ray diagnostic installation according to Claim 3, characterized in that the housing (8) is provided on the base side with a drawer (34), which can be withdrawn or folded down, for holding accessories.

7. Dental X-ray diagnostic installation according to Claim 3, characterized in that the housing (8) is provided with a bearing means (25) for swivellably holding an X-ray machine (15) for producing intra-oral images.

8. Dental X-ray diagnostic installation according to Claim 7, characterized in that a common patient's chair (12), which is supported by a swivelling arm (13) mounted on the base side, is provided for the X-ray machine for producing the intra-oral images, and for the X-ray diagnostic installation for producing the panoramic X-ray images.

9. Dental X-ray diagnostic installation according to Claim 8, characterized in that a common control part (35) is present for the purpose of driving the X-ray sources (5, 18) of the X-ray machine and the X-ray diagnostic installation.

10. Dental X-ray diagnostic installation according to Claim 9, characterized by the presence of a changeover switch (46) by means of which an electronic control unit (36) can change over to one (5) or the other (18) of the X-ray sources as a function of the position of the devices.

11. Dental X-ray diagnostic installation according to one of Claims 1 to 10, characterized in that the positioning unit (9) contains a head holder (19) of visor-like construction, on which there are arranged an adjustable forehead support (20), temple supports (21) which can be placed against the patient's head, and a bite-down part (22) for fixing the front teeth of the patient.

12. Dental X-ray diagnostic installation according to one of Claims 1 to 11, characterized in that the X-ray machine for producing the intra-oral images contains a digital image pick-up and a corresponding image evaluation device, and the housing (8) is the support for an input and/or image-reproducing device (48, 49).

13. Dental X-ray diagnostic installation according to Claim 4, characterized in that arranged securely in terms of rotation, but in axially displaceable fashion on the swivelling shaft (7) is one part (31b) of a coupling (31) whose other part (31a) is connected to the positioning device (9).

## Revendications

1. Dispositif de diagnostic radiologique dentaire destiné à l'établissement de radiographies panoramiques du crâne d'un patient, comportant une unité rotative (1) pouvant être déplacée autour de la tête du patient, sur laquelle une source (5) de rayons X et une unité de radiographie (6) sont fixées diamétralement à l'opposé l'une par rapport à l'autre, ainsi qu'une unité de positionnement (9) qui immobilise la tête du patient de façon reproductible, l'unité rotative (1) et l'unité de positionnement (9) étant disposées en console sur un support, et l'unité rotative pouvant être pivotée autour d'un axe horizontal et bloquée dans différentes positions, caractérisé en ce que l'unité rotative (1) et l'unité de positionnement (9) peuvent être pivotées séparément autour d'un palier commun dont l'axe de pivotement (7) est parallèle au plan de l'unité rotative (1), et sont disposées de façon à pouvoir être respectivement bloquées dans différentes positions pivotées, les différentes positions pivotées correspondant à différentes tailles d'un patient, de préférence assis, par le fait que l'unité de positionnement (9) peut dans un premier temps être réglée par rapport au patient, et que l'unité rotative (1) est ensuite réglée en association fixe avec l'unité de positionnement (9).

2. Dispositif de diagnostic radiologique dentaire selon la revendication 1, caractérisé en ce que l'unité rotative (1) peut être déplacée d'une position de non utilisation, dans laquelle la source (5) de rayons X et l'unité de radiographie (6) sont situées dans un plan sensiblement vertical et parallèle à l'unité rotative (1), dans plusieurs positions d'utilisation, dans lesquelles le plan formé par la source (5) de rayons X et l'unité de radiographie (6) forme un angle aigu par rapport au plan vertical précité.

3. Dispositif de diagnostic radiologique dentaire selon la revendication 1, caractérisé en ce que l'axe de pivotement (7) est monté dans un boîtier (8) pouvant de préférence être fixé sur un mur, dans lequel sont logés des moyens destinés à l'activation de la source (5) de rayons X et à la commande du mouvement de l'unité rotative (1).

4. Dispositif de diagnostic radiologique dentaire selon la revendication 1, caractérisé en ce que l'unité rotative (1) est reliée solidairement à l'axe de pivotement (7), et en ce que ce dernier est monté en rotation dans le boîtier (8).

5. Dispositif de diagnostic radiologique dentaire selon la revendication 4, caractérisé en ce que l'axe de pivotement (7) est muni d'un ressort de torsion (28) destiné à la compensation du poids de l'unité rotative (1), lequel prend appui, d'une part sur l'axe de pivotement (7), et d'autre d'autre part sur le boîtier (8).

6. Dispositif de diagnostic radiologique dentaire selon la revendication 3, caractérisé en ce que le fond du boîtier (8) est muni d'un compartiment (34) rabattable ou pouvant être retiré, qui est destiné au rangement d'accessoires.

7. Dispositif de diagnostic radiologique dentaire selon la revendication 3, caractérisé en ce que le boîtier (8) est muni d'un moyen de logement (25) pour la fixation pivotante d'un appareil de radiologie (15) destiné à l'établissement de radiographies aux infrarouges.

8. Dispositif de diagnostic radiologique dentaire selon la revendication 7, caractérisé en ce que, pour l'appareil de radiologie destiné à l'établissement de radiographies aux infrarouges et pour le dispositif de diagnostic radiologique destiné à l'établissement de radiographies panoramiques, on prévoit une chaise (12) de patient commune qui est portée par un bras pivotant (13) fixé au sol.

9. Dispositif de diagnostic radiologique dentaire selon la revendication 8, caractérisé en ce qu'un élément de commande commun (35) est prévu pour l'activation des sources (5, 18) de rayons X de l'appareil de radiologie et du dispositif de diagnostic radiologique.

10. Dispositif de diagnostic radiologique dentaire selon la revendication 9, caractérisé en ce qu'on prévoit un inverseur (46), avec lequel une électronique de commande (36) peut être commutée en fonction de la position des appareils sur une source (5) de rayons X ou sur l'autre (18).

11. Dispositif de diagnostic radiologique dentaire selon l'une quelconque des revendications 1 à 10, caractérisé en ce que l'unité de positionnement (9) comporte un support de tête (19) de type visière, sur lequel sont disposés un support de front (20) réglable, des supports de tempes (21) pouvant être appliqués sur la tête du patient, ainsi qu'un élément de mors (22) destiné à immobiliser les incisives du patient.

12. Dispositif de diagnostic radiologique dentaire selon l'une quelconque des revendications 1 à 11, caractérisé en ce que l'appareil de radiologie destiné à l'établissement de radiographies aux infrarouges comporte un dispositif de prises de vue numérique et un dispositif d'évaluation d'image correspondant, et en ce que le boîtier (8) porte un dispositif d'entrée et/ou de restitution d'image (48, 49).

13. Dispositif de diagnostic radiologique dentaire selon la revendication 4, caractérisé en ce qu'une partie (31b) d'un couplage (31) est disposée solidairement mais de façon axialement mobile sur l'axe de pivotement (7), l'autre partie (31a) de ce couplage étant reliée à l'unité de positionnement (9).
